# EUROPEAN PATENT APPLICATION

(11) **EP 0 875 223 A2**
(43) Date of publication of application: **04.11.1998**
(21) Application number: 98107332.3
(22) Date of filing: 22.04.1998
(51) Int. Cl.: A61F 13/06, A61F 13/10, A61F 13/12, A61F 13/14

(54) **Myofascial bandage**

(30) Priority: 30.04.1997 IT PD970095
(71) Applicant: Martella, Pasquale, 35010 Vigodarzere (Padova) (IT)
(72) Inventor: Martella, Pasquale, 35010 Vigodarzere (Padova) (IT)
(74) Representative: Modiano, Guido, Dr.-Ing.

(57) **Abstract**

A myofascial bandage constituted by a band (10) of stretch fabric whose ends are provided with means (11a, 11b) for mutual engagement when subjected to traction. The band (10) has at least one pocket (12, 13; 17, 18) in which a spindle (14; 19, 20) shaped like an ellipsoid of revolution is inserted; the spindle applies a variable compressive action to the skin, to the fascia and in the grooves between the muscles located in the area where it is placed.

## Description

The present invention relates to a myofascial bandage for treating inflammatory and/or posttraumatic pains.

It is known that in the human body, muscles have a continuity and contiguity relationship with connective-type structures such as fasciae, tendon sheaths, tendons, synovial bursas and ligaments.

Connective tissue is constituted by fibroblasts, elastic fibers and collagen fibers arranged in bundles and by lacunar liquid.

This liquid constitutes the interstitial lymph, which is the point of origin of lymphatic vessels and whose movement is linked to the movement of the fascia.

The common fascia or superficial aponeurosis covers almost all of the body surface, covers the surface skeletal muscles and blends with their perimysium and in some points splits into two sheets in order to envelope them.

Septa known as intermuscular septa originate from the fascia and connect to the periosteum and the main ones divide the muscles into compartments.

The aponeurosis has variable thicknesses depending on whether it envelopes or acts as an attachment point for the fleshy bundles of muscles, has a small number of skeletal attachment points and some of said points are located on movable bones.

The aponeurosis starts from the cranium and descends in the posterior region along the spine and attaches to the spinous processes and in the anterior region along the breastbone and along the linea alba; at the upper limbs it is fixed to the clavicula, to the scapula and to the posterior rim of the ulna; at the lower limbs, it is fixed to the anterior internal face of the tibia, to the calf bone, to the lateral rims of the patella, to the patellar tendon and to the anterior tuberosity of the tibia.

This system conveys the impression of unity and therefore a mechanical alteration linked to dysmorphia, to a trauma (even a minor one) or to an inflammatory process affects the entire system.

According to the prior art, inflammatory or posttraumatic syndromes are currently cured by using, according to the regions, simple bands, bands with splints, knee-bands, corsets with metal rods or collars combined with physical therapy.

All these systems have been found to be scarcely suited to the treatment of the different syndromes and require very long recovery times.

Additionally, the different devices are also uncomfortable and poorly tolerated.

The aim of the present invention is to provide a myofascial bandage which solves the problems of treating inflammatory and/or posttraumatic syndromes.

This aim and other objects which will become apparent hereinafter are achieved by a myofascial bandage, characterized in that it is constituted by a band which has mutual engagement means at its ends and has, in an intermediate region, at least one pocket containing a spindle which applies a localized pressing action to the body region where the band is applied.

Further characteristics and advantages of the present invention will become apparent from the following detailed description of some embodiments thereof, illustrated only by way of non-limitative example in the accompanying drawings, wherein:
Figure 1 is a perspective view of a bandage according to the present invention provided with two spindles;
Figure 2 is a schematic view of the application of the bandage to a knee; and
Figure 3 is a perspective view of a soft cervical collar provided with the spindles according to the present invention.

With reference to the above figures, the invention is composed of a band 10 made of stretch or double-stretch material of appropriate length which has, at its ends, mutual engagement means 11a and 11b which in the illustrated case are constituted by two complementary elements made of Velcro.

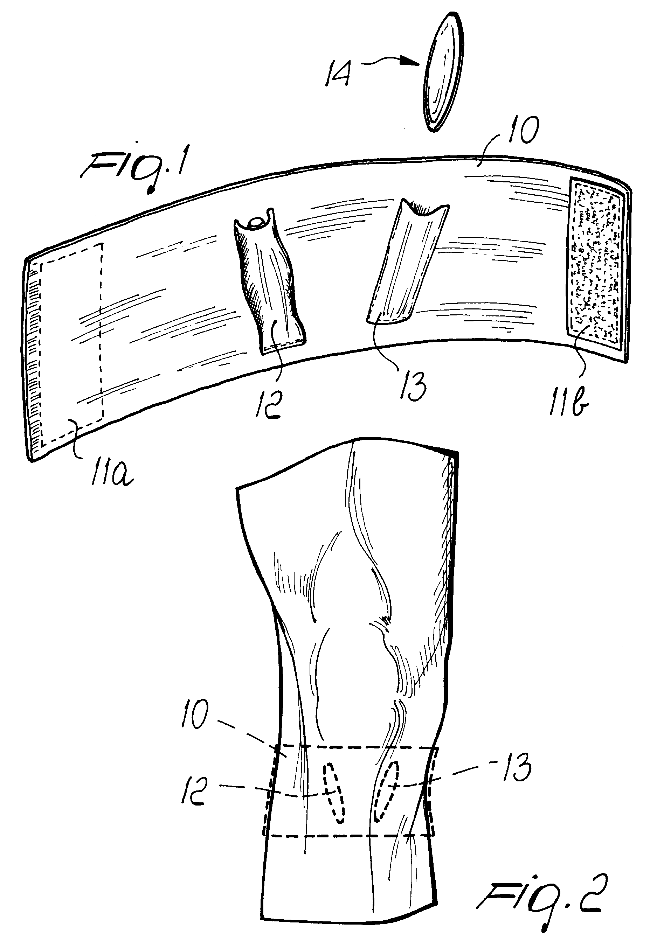

In the example shown in Figure 1, two pockets, designated by the reference numerals 12 and 13 respectively, are formed on said band 10 by sewing pieces of fabric.

A spindle 14 is inserted in each one of the two pockets 12 and 13 and is constituted by a body which is shaped like an ellipsoid of revolution made of a flexible material.

Preferably, the spindle 14 is made of blow-molded PVC, which forms a hollow structure.

A solid or liquid mass can be placed in the cavity of the spindle and produces partial filling.

As the person moves, this unrestrained mass also moves, generating vibrations of the spindle 14 stimulating the myofascial tissue.

The same spindle 14 can be obtained, as an alternative, from a spongy and elastic plastic material.

Conveniently, the dimensions of the spindle 14 are:
-- length: 5.5 cm
-- maximum diameter in the central region: 1.4 cm
-- diameter in the region proximate to the rounded ends: 0.6 cm.

The bandage shown in Figure 1, as shown in Figure 2, is suitable for treating knee syndromes; accordingly, the two pockets 12 and 13 are arranged so as to mutually diverge upward.

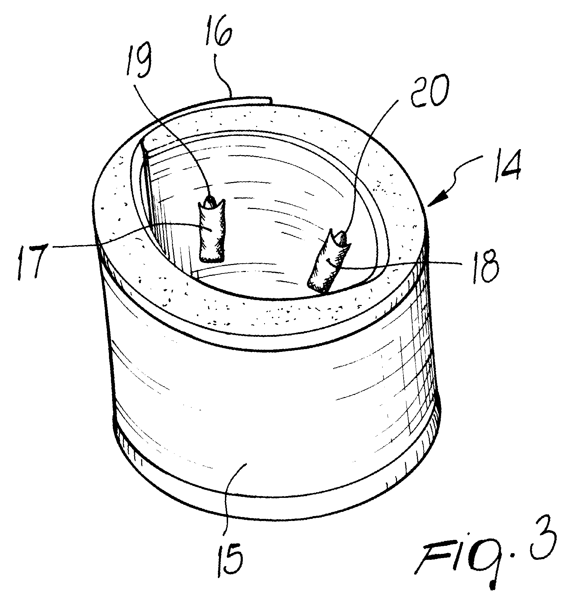

Figure 3 is a view of a cervical collar, generally designated by the reference numeral 14, which is provided by means of a semirigid foam rubber structure 15 covered by a stretch-type tubular mesh and provided with a Velcro closure 16.

In the internal part there are provided two pockets 17 and 18 containing two spindles 19 and 20 of the same type already described.

In this case too, the two spindles 19 and 20 can be made of blow-molded PVC or elastic sponge.

The mechanism of action of the myofascial bandage according to the invention is evident from the above description.

The spindle applies a variable compressive action to the skin, to the fascia and between the muscles, depending on the mechanical gestures performed in everyday life.

This action allows an activation of the proprioceptor nervous circuits by means of a continuous stimulation of the mechanoreceptors and allows the muscles greater extension, so that the gesture is executed as correctly as possible.

The spindle also performs a particular kind of massage (compressive friction) which, by stimulating blood circulation, carries oxygen to the cells of the treated region and facilitates venous and lymphatic flow, allowing reabsorption of oedemae.

Compressive friction applied to the muscle fibers increases fiber contractility and improves their microcirculation.

The result of all this is muscular relaxation, with an increase in tonicity, trophicity and mobility.

Another action of the spindle is to allow better mutual sliding of the tissues; this is an important factor for the elimination of muscle contractures and tensions.

All the actions performed by the spindle achieve the aim of returning to mobility of the articular strokes.

Some methods of application are described by way of example.

### SHOULDER BANDAGE

The spindle must be placed in the external bicipital groove, located on the front face of the shoulder, which separates the deltoid muscle from the brachial bicipital muscle.

In these conditions the spindle is suitable for treating anterior acute shoulder pain, global acute shoulder pain, anterior chronic shoulder pain and algodystrophic shoulder pain, and is an adjuvant in rehabilitation after surgery for acromiohumeral conflict syndrome and rotator cuff rupture, an adjuvant in physical reeducation following shoulder dislocation and fracture of the upper third of the humerus.

### ELBOW BANDAGE

The spindle must be arranged in the longitudinal groove that separates the prominence of the musculus brachioradialis and the venter of the extensores carpi radiales from the flat area of the extensor digitorum.

In this application the spindle is suitable for the treatment of epicondylitides and is an adjuvant in the rehabilitation of traumatic disorders of the elbow.

### WRIST BANDAGE

This bandage allows to treat various disorders by diversifying the position of the spindle.

In tendinitis of the wrist extensors, the spindle must be arranged in the median region of the dorsal face of the distal third of the forearm.

In De Quervain's disorder, the spindle is arranged in the radial region of the dorsal face of the distal third of the forearm.

In tendinitis of the wrist flexors, the spindle is placed in the median region of the palm side of the distal third of the forearm.

In fractures of the lower ends of the radius or scaphoid and in various kinds of wrist dislocation, the spindle must be arranged alternately in the median region of the palm and dorsal face of the distal third of the forearm.

### KNEE BANDAGE

The ischiocrural muscles diverge on the posterior face of the knee, forming a triangle.

The medial side is formed by the semitendinous and semimembranous muscle and the lateral side is formed by the musculus biceps femoris.

The bandage has, in this case, two spindles which diverge from above and rest as follows: the medial one rests laterally to the venter and the tendons of the semimembranous and semitendinous muscle; the lateral one rests laterally to the venter and the tendon of the musculus biceps femoris.

Indications for treatment of this bandage are knee arthrosis, while the bandage is an adjuvant in the physical reeducation of fractures of the lower third of the femur and of the tibial plate, in sprain-like traumas of the knee, in tendinitis of the biceps femoris and in tendinitis of the semimembranous muscle.

### PATELLAR BANDAGE

The two spindles converge downward and are arranged on the two sides of the patellar tendon.

This bandage is indicated for treatment of rotular chondromalacia, femoral-patellar arthrosis, Osgood-Schlatter disease, insertional tendonopathy of the patellar tendon, patellar fracture, osteochondritis of the lower apex of the patella.

### ACHILLES TENDON BANDAGE

The two spindles converge downward and are arranged on either side of the Achilles tendon.

This bandage is indicated for treating achillotendonopathy, retrocalcaneobursitis, plantar fascitis, physical reeducation following rupture of the Achilles tendon and calcaneal apophysitis.

### FASCIAL BANDAGE - ANTERIOR TIBIAL SYNDROME

Anterior tibial syndrome and medial tibial syndrome are ischemic vascular syndromes caused by an increase in muscle mass with respect to the extensibility of the fascia.

In anterior tibial syndrome, the spindle is placed between the anterior margin of the tibia and the anterior tibial muscle at the upper third of the leg.

In medial tibial syndrome, the spindle is arranged in the medium-lower third of the leg between the medial margin of the tibia and the peroneal muscles.

### LUMBAR BELT

The belt consists of six spindles arranged in a checker-like pattern to the sides of the paravertebral muscle masses of the lumbar spine.

This bandage, which has the appearance of a corset, is adapted for acute backache, chronic backache, osteoporosis of the spine, occasionally in spondylolysis and spondylolisthesis, in rehabilitation treatment for slipped-disk surgery and vertebral instability and following fracture of the lumbar spine.

### PUBALGIA FASCIAL BANDAGE

The spindle is arranged in the medial face of the middle third of the thigh.

This bandage is suitable for curing pubalgia.

### CERVICAL COLLAR

In this case there is provided a semirigid foam rubber structure covered with stretch-type tubular mesh and closed for example with Velcro, which has, in the inner part, two spindles which are arranged respectively to the right and to the left in the groove that separates the trapezius muscle from the sternocleidomastoid muscle.

This collar is suitable for curing acute cervicalgia, cervical arthrosis, torticollis, spinal-cervical whiplash.

From the above description and the illustrations it is evident that the intended aim and all the objects have been achieved by providing a myofascial bandage which, although being simple in its embodiment, has been proved by experimental tests to be highly effective in the described treatments.

The materials and the dimensions may of course be any according to requirements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A myofascial bandage, characterized in that it is constituted by a band provided, at its ends, with mutual engagement means and has, in an intermediate region, at least one pocket containing a spindle which applies a localized compressive action to the body region where the band is applied.

2. A bandage according to claim 1, characterized in that said band is at least partially made of stretch and/or double-stretch fabric.

3. A bandage according to claim 1, characterized in that the mutual engagement means of the ends of the band are constituted by Velcro.

4. A bandage according to claim 1, characterized in that said spindle is shaped like an elongated ellipsoid of revolution.

5. A bandage according to claim 4, characterized in that said spindle is made of plastics, particularly PVC, and is obtained by blow-molding and is hollow and elastic.

6. A bandage according to claim 5, characterized in that a solid or liquid mass is inserted in said hollow spindle, partially fills the cavity of the spindle, is free to move and generates vibrations stimulating the myofascial tissue.

7. A bandage according to claim 4, characterized in that said spindle is made of spongy and elastic plastic material.

8. A bandage according to claim 4, characterized in that said spindle is 5.5 cm long, has a diameter of 1.4 cm in the central part and a diameter of 0.6 cm in the region proximate to the rounded ends.

9. A bandage according to claim 1, characterized in that said pocket and said spindle are arranged at right angles to the main dimension of the band.

10. A bandage according to claim 1, characterized in that said pocket and said spindle are arranged at an angle with respect to the main dimension of said band.

11. A bandage according to claim 1, characterized in that it has a plurality of pockets in which a plurality of spindles are inserted, said spindles being parallel or inclined with respect to each other.

12. A bandage according to claim 1, characterized in that it is arranged in a body region requiring compressive stimulation for curing inflammatory and/or posttraumatic pains.
